Europäisches Patentamt
European Patent Office
Office européen des brevets

Publication number: **0 367 283 A2**

# EUROPEAN PATENT APPLICATION

Application number: 89120384.6

Date of filing: 03.11.89

Int. Cl.[5] C08G 18/48 , C08G 18/40 , C08G 18/65 , //(C08G18/48, 101:00)

Priority: 04.11.88 US 267201

Date of publication of application: 09.05.90 Bulletin 90/19

Designated Contracting States: AT BE CH DE ES FR GB GR IT LI LU NL SE

Applicant: UNION CARBIDE CHEMICALS AND PLASTICS COMPANY INC. (a New York corporation)
39 Old Ridgebury Road
Danbury Connecticut 06817-0001(US)

Inventor: Whitman, Robert Don
1620 Kirklee Road
Charleston West Virginia, 25314(US)
Inventor: Argento, Benny John
1203 Crown Drive
South Charleston West Virginia, 25309(US)

Representative: Wuesthoff, Franz, Dr.-Ing. et al
Patentanwälte Wuesthoff & Wuesthoff
Schweigerstrasse 2
D-8000 München 90(DE)

## Wet set additives for high resilience foam.

Ethylene oxide (EO) adducts of di- ard tri-functional starters, such as, trimethylolpropane and glycerine, provide unexpected improvements in the "wet compression set" properties of high resilience (HR) urethane foams. Heretofore, wet set improvements have been achieved through the use of EO adducts of tetrols because of the high degree of crosslink density which these materials impart to a urethane polymeric composition. One commercial wet set additive, which is an EO adduct of pentaerythritol and diglycerine, is an effective wet set improver. Surprisingly, EO adducts of di- and tri-functional starters provide improvements in foam wet set properties, which are comparable to those obtained with such commercial wet set additive. Specifically, EO adducts of glycerine in the 500 to 10,000 molecular weight range yield foam wet set improvements equal to those of such commercial wet set additive at as low as 20 percent of the use level on an equivalent weight basis. Moreover, the EO adducts of glycerine yield foams having improved elongation properties and improved processing (stability) characteristics as compared to those observed with such commercial wet set additives. An EO adduct of glycerine having a number average MW of 1,000 is particularly useful in the invention.

EP 0 367 283 A2

Xerox Copy Centre

# WET SET ADDITIVES FOR HIGH RESILIENCE FOAM

## 1. Field Of The Invention

The invention relates to wet set additives for the producing of high resilience (HR) polyurethane foam products, for example, in automotive seating, furniture, bedding and other products, to resin blends to make such high resilience polyurethane foams, to foam cushions and other foam products composed of such HR polyurethane foams, and to processes of preparing such HR polyurethane foam products.

## 2. Prior Art

High resilience polyurethane foams are sometimes hereinafter referred to as HR foams. Japanese automobile companies employ HR molded foams and Hot molded foams in their seating applications. HR foams are used in cushions and Hot foams are used primarily in seat backs. The HR cushion foam technology which they use is similar to that of the U.S. automobile industry, but their densities are generally much higher (50 to 100 percent higher). Their foam property specifications are like those in the U.S., except for one additional property which they judge to be very important to good seat cushioning performance. This property is "wet compression set" and it usually referred to by the simple phrase "wet set."

The wet set property is determined as the permanent set after a 30-minute recovery from 22 hours of 50 percent compression at conditions of 50°C and 95 percent R.H. This test simulates the effects of high humidity on seat cushion properties in automobiles exposed to tropical and sub-tropical environments. The Japanese automotive industry considers the test to be a critical part of their performance specifications for automotive seating for Far Eastern countries.

Japanese automotive transplants in the U.S. have adopted the wet set standard for their automotive seating foams, particularly in meeting their established wet set specifications in HR foam front and rear seat cushions.

Most automobile manufacturers supply foam elongation values of 120 percent or greater. This presents a real problem since many factors that improve wet sets also tend to decrease elongation values. Increased foam density is a known way of improving both wet set and elongation properties. However, this is unattractive due to the economic penalty occurred.

The use of additives to improve wet set properties of low density foam is highly desirable from a cost/performance standpoint.

Mitsui Toatsu Chemicals Inc. markets such an additive, identified as KL-200, which is effective in improving HR foam wet set properties. This material appears to function as a crosslinker in the urethane polymer. The additive, KL-200 is an ethylene oxide adduct of 80/20 pentaerythritol/diglycerine (a tetra-functional starter) to a hydroxyl number of 550 (molecular weight of 102). The major drawbacks of using KL-200 are: (1) the foam elongation values are adversely affected; and (2) foam instability is induced with its use. KL-210 is a mixture of KL-200 and diethanolamine.

JP-AL- 63-75021 discloses ethylene oxide adducts of tetrol starters for HR polyurethane foams, including KL-200 and KL-210.

Saotome et al. "Journal Of Cellular Plastics", (May/June 1977), pp. 203 to 209, is a survey paper dealing with the improvement of the humidity resistance of high resilience polyurethane foam.

GB- 2,185,032 uses an ethylene oxide-propylene oxide-trimethylolpropane adduct and cites compression set values after the polyurethane foam is humid-aged.

## BROAD DESCRIPTION OF THE INVENTION

An object of the invention is to improve the "wet compression set" properties of high resilience (HR) polyurethethane foams. Another object of the invention is to provide wet set additives for HR polyurethane foams. A further object of the invention is to provide preblends for the prepartion of HR polyurethane foams. A still further object of the invention is to provide processes for preparing HR polyurethane foams. Another object of the invention is to provide HR polyurethane foams for automotive seat cushions and the like which have improved wet set values without significant reduction of elongation and load bearing values. Other objects and advantages of the invention are set out herein or are obvious herefrom to one skilled in the art.

The invention involves a composition for use in the preparation of high resilience polyurethane foam. The invention composition includes: an ethylene oxide adduct of a trifunctional starter compound or a difunctional starter compound; and a polyol, provided that the polyol is not the same as the adduct. The invention composition also includes a catalyst, water and at least one foam stabilizer. This composition is reacted with an organic polyisocyanate. The preferred additive is an ethylene oxide adduct of glycerine to a number average MW of about 1,000. The invention compositions are liquids, solutions or dispersions. The adduct can be liquid or solid; if it is solid, it can be dissolved or dispersed in one of the major liquid components of the composition. The use of the ethylene oxide adduct of a di- or trifunctional starter results in better elongation and processing properties than achieved with higher functional additives such as KL-200, while securing HR polyurethane foams with equal or comparable wet compression set.

The invention involves processes for preparing the high resilience foam.

The invention also involves a process for producing a high-resilience molded polyurethane foam. The process includes:

(a) mixing the urethane chemicals of the invention;
(b) pouring the chemical mixture into a mold;
(c) allowing the foam to expand and cure; and
(d) removing the foam from the mold.

The invention further includes the high resilience polyurethane foams, including in article form such as those obtained from molding and slab stock processes.

It has been discovered that ethylene oxide adducts of a triol starter, preferably glycerine are more effective wet set improvers in HR foams than those made with a tetrol starter, on an equal equivalent weight basis. (The same results are secured with a diol, diamine or triamine starter.) This is quite unexpected since the triol started material is not as effective as a crosslinker as the tetrol. (Since a diol starter gives similar results, crosslinking is apparently not the mechanisms of the invention adducts.) A second unexpected result of the use of triol additives is that a significant improvement in foam elongation properties are achieved in foams having equal wet set imrovements. (The same results are secured with a diol, diamine or triamine starter.) Furthermore, the triols appear to cause much less foam instability than that observed with the tetrols.

Ethylene oxide adducts of trimethylolpropane starter have also been found to be equally as effective as glycerine started materials.

The polyurethane foams of the invention have a high resilience and excellent cushioning properties. The polyurethane foams of the invention can be used as seat cushioning material for vehicles, aircraft, furniture and the like.

The preferred mode of practicing the invention involves the following.

- High molecular weight polyols (>4500) containing more than 10 percent of ethylene oxide and more preferably more than 15 percent. The primary hydroxyl content should be greater than 80 percent.
- Polymer polyols based on the above to control the load bearing properties of the foam. Preferably these are based on the in situ polymerization of a vinyl monomer in the polyol, but other dispersion polyols are also useful in the invention.
- Water levels greater than 2 parts per hundred parts of polyol.
- Tolylene diisocyanate or mixtures of tolylene diisocyanate and polymethylene polyphenyl isocyanates at 95 to 115 index (stoichiometry).
- Tertiary amine catalysts, such as NIAX® catalyst A-1, for the blowing reaction. Acid blocked catalyst can be used to achieve rapid demold foam.
- Tertiary amine catalyst, such as NIAX® catalyst A-33, for the gelatin reaction. Acid blocked catalyst can be used to achieve rapid demold foam.
- An alkanolamine, such as diethanolamine, to stabilize all-tolylene diisocyanate foams. An 80/20 mixture of tolylene diisocyanate and polymethylene polyphenyl isocyanates requires no alkanolamine stabilizer. High molecular weight, high resilience polyurethanes need a foam stabilizer.
- Wet set additive (ethylene oxide adduct of a triol starter) having a number average MW of about 1,000, in the range of 1 to 6 parts per hundred parts of polyol, more preferably about 4 parts.
- A silicone surfactant such as Union Carbide Corporation's silicone Y-10,515. The silicone surfactants are also foam stabilizers.

This combination of components yields a high quality, medium to high density HR foam meeting critical wet set requirements with improved elongation properties.

Combinations of various molecular weights of triol or diol additives can offer further improvements to this technology.

Ethylene oxide adducts of diol starters, such as ethylene glycol, also function as wet set improvers.

KL-200 of Mitsui Toatsu Chemicals, Inc. apparently acts in a crosslinking manner. This material has very adverse effects on foam stability and elongation properties and, therefore, they must be used sparingly.

The experimental data has shown that the improvement achieved by the invention is not caused by increased crosslinking of the polyurethanes because the invention improvements are also achieved by the use of difunctional starters.

The use of ethylene oxide adducts of di- and trifunctional starters improves the foam wet set properties of high resilience molded polyurethanes at least as much as does KL-200. The invention wet set additives were found to be the most effective cushion formulation component to improve foam wet sets without sacrifices in density or cure rates. A low molecular weight ethylene oxide adduct of glycerine having a number averge MW of 1,000 to be optimum for this use. Only a moderate (10 to 15 percent) reduction in elongation results from the utilization of the maximum effective level of such additive.

The invention molded polyurethanes equal the wet set properties of those produced using KL-200, have better mechanical properties and involve lower costs. The invention polyurethane foams have better elongation and load bearing properties (the reduction of these properties is less than with KL-200). Improvement of wet set values results in reduction of elongation and load bearing values, but the invention has minimized the reduction of the elongation and load bearing values.

## DETAILED DESCRIPTION OF THE INVENTION

The present invention is especially suited to the manufacture of high resilience polyurethane foamed products, such as, seat cushions for automobile and trucks. The foamable reaction mixture is conveniently prepared by mixing all of the ingredients with a polyisocyanate and then quickly introduced into the mold. The mold is then closed. The ingredients are composed of the polyol which can be a polymer/polyol or a mixture of a polyol and a polymer/polyol; water; a catalyst or catalysts; a surfactant or surfactants; at least one low molecular weight ethylene oxide adduct of a difunctional or trifunctional starter compound; and in addition any other ingredients such as pigments or special effects additives. Since none of these ingredients inter-react with each other they can be mixed well in advance of adding the polyisocyanate. However, once the polyol and/or water are contacted with the polyisocyanate, the polyurethane-forming or $CO_2$ forming reaction begins. If auxiliary blowing agents are utilized, such as fluorocarbons, they are quite volatile and it is preferred to add them to the other ingredients just prior to when it is to be used.

The suitable trifunctional starter compounds and difunctional starter compounds, for use as wet set additives, have three active hydrogens and two active hydrogens, respectively. Examples of such functional groups having active hydrogens are hydroxyl and amine groups, so the invention starters include amino and hydroxyl (di and tri) functional compounds. The preferred functional groups are hydroxyl groups. As used herein, the phrase "active hydrogen-containing starter compound" refers to compounds containing two or three active hydrogen-containing groups each of which is capable of reacting with ethylene oxide. For purposes of the invention, such active hydrogen-containing compounds are compounds containing two or three active hydrogens as determined by the well known Zerewitinoff test such as described by Kohler in Journal Of The American Chemical Society, 49, 3181 (1927). The di- and trifunctional starters preferably have active hydrogen-containing groups at their terminus.

The non- di- or trifunctional starter portion of the adduct should be predominantly ethylene oxide (as opposed to other alkylene oxides, for example).

As used herein, the term triol includes many organic compounds having three hydroxyl groups on an essentially organic substrate and the term diol includes many organic compounds having two hydroxyl groups on an essentially organic substrate. The diols and triols can be aliphatic compounds, aryl compounds including aromatic compounds, and heterocyclic compounds. Primary, secondary and/or tertiary diols and/or triols can be used.

Any suitable trifunctional starters can be used to prepare the ethylene-oxide adducts of trifunctional starters. The most preferred trifunctional starter is glycerine. A preferred trifunctional starter is 1,1,1-trimethylolpropane.

Hydroxyl groups, for example, are suitable functional groups in the trifunctional starters. Examples of triol or trihydric alcohol (trifunctional) starters are the alkanetriols (e.g., $C_2$ to $C_{10}$), such as, 1,1,2-ethanetriol, 1,2,4-butanetriol 1,1,1-trihydroxymethylethane, 1,2,3-butanetriol(1-methylglycerol), 1,2,4-butanetriol, 1,2,7-heptanetriol, 1,2,5-hexanetriol, 1,2,8-octanetriol, 1,2,9-nonane triol, 1,2,10-decanetriol, 1,2,6-hexanetriol, and 1,1,1-methylolethane, and the cycloalkyl triols, such as, 1,3,4-cyclohexane triol.

The trifunctional starter compounds can be aromatic triols (aromatic trihydric alcohols), such as, trihydric phenols, for example pyrogallol (1,2,3-trihydroxybenzene), phloroglucinol (sym-trihydroxybenzene) and hydroxquinol (1,2,4-trihydroxybenzene), 2,4,5-trihydroxytoluene, 2,4,6-trihydroxytoluene and 2,3,5-trihydroxytoluene. Mixed aliphatic aromatic compounds (aromatic alcohols) which have three hydroxyl groups each attached to a side chain or aliphatic portion thereof can be used.

The difunctional starter compounds can be diols (dihydric alcohols), such as aliphatic diols, for example, ethylene glycol, propylene glycol, 1,3-propanediol, 1,2-butanediol, 2,3-butanediol, 1,3-butanediol, 1,4-butanediol, isobutylene glycol, 1,5-pentanediol, tetramethylglycol, 2-methyl-2,4-pentanediol, 1,12-octadecanediol, 3-methyl-1,2-butanediol, 1,2-propanediol, 1,3-propanediol, 1,4-pentanediol, 2,2-dimethyl-1,5-pentanediol, 1,2-hexaneglycol, 1,6-hexanediol, 1,7-heptanediol, 1,8-octanediol, 1,2-butyleneglycol, 1,9-nonanediol, 1,10-decanediol, neopentyl glycol, 1,4-bis-hydroxylmethylcyclohexane, butadiene diol, butylene glycol and diethylene glycol, dihydric phenols, for example, pyrocatechol (o-dihydroxybenzene), resorcinol (m-dihydroxybenzene), hydroquinone (p-dihydroxybenzene), dihydric toluenes, for example, 2,3-dihydroxytoluene, 2,4-dihydroxytoluene, 3,5-dihydroxytoluene, 3,4-dihydroxytoluene, 2,5-dihydroxytoluene, other aryl diols, for example, 1,2-dihydroxynepthalene, 1,4-dihydroxynepthalene and 2,2-bis(4-hydroxyphenyl)-propane, heterocyclic diols, cycloalkyl diols, for example 1,4-cyclohexane diol and 1,2-cyclohexane diol, and other diols, for example, 3-cyclohexane-1,1-dimethanol.

Straight chained or branched alipahtic diols with up to 12 carbon atoms are particularly useful. The diols can be alkylene diols of the formula $HO \cdot (CH_2)_n \cdot OH$ wherein n is an integer of 2 to 12. The diols can be polyalkylene oxide glycols having the formula $HO(RO)_nH$, wherein R is an alkylene radical and n is an intefer greater than 1. The diols can also be polyalkylene glycols having the formula $HO(CH_2OC_2H_4O)_nH$, wherein n is an integer of 1 or more.

The trifunctional starter compounds can be trifunctional starter compounds having combinations of amino and hydroxy groups in the total amount of three active hydrogens. Examples of the trifunctional amino/hydroxy starter compounds are the trifunctional amino/hydroxy alkanes, for example, diethanolamine, the trifunctional amino/hydroxy aryl compounds, for example, ortho, meta or para-aminophenol, and the trifunctional amino/hydroxy cycloalkyl compounds, for example, 1,6-aminocyclohexanol.

Any suitable ethylene-oxide adducts of trifunctional starter compounds can be used. The most preferred ethylene-oxide adducts of trifunctional starters are the ethylene-oxide adducts of glycerine (glycerol). Preferably the ethylene oxide adduct compounds have an average molecular weight of 500 to 10,000, particularly preferably 700 to 4,500. An ethylene oxide adduct of glycerine having a number average molecular weight of about 1000, is most preferred. Other useful examples are an ethylene oxide adducts of glycerine having a number average molecular weight of 2000, 4000 or 6000.

The ethylene-oxide adducts of trimethylolalkanes are particularly useful, the ethylene-oxide adducts of trimethylolpropane being preferred.

The ethylene-oxide adducts of tritunctional starters preferably have an average molecular weight of 500 to 10,000.

The invention does not include tetrafunctional compounds, such as, tetrols and tetramines, or higher polyfunctional compounds.

Any suitable process can be used to prepare the di-and trifunctional starters. Typically, 2 to 200 moles of ethylene oxide can be added to the di- or trifunctional starter.

The following deals with the other essential ingredients of this formulation.

## POLYOLS

The polyol, or polyether polyol, or blends thereof, employed depends upon the end use of the polyurethane foam to be produced. The molecular weight or hydroxyl number of the base is selected so as to result in flexible foams when the polyol is converted to a polyurethane. For the purpose of the present invention for polyols are characterized by having at least 75 percent, and preferably 90 percent, primary hydroxyl groups as measured by ASTM D-4273. The hydroxyl number of the polyols employed can accordingly vary over a wide range. In general, the hydroxyl number of the polyols employed may range from 20 (or lower) to 70 (or higher). As a further refinement, the specific foam application will likewise influence the choice of the polyol. As an example, for molded foam, the hydroxyl number of the polyol may be on the order of 20 to 40, and for slabstock the hydroxyl number may be on the order of 25 to 70.

The hydroxyl number limits described above are not intended to be restrictive, but are merely illustrative of the large number of possible combinations for the polyols used.

The hydroxyl number is defined as the number of milligrams of potassium hydroxide required for the

complete hydrolysis of the fully phthalated derivative prepared from one gram of polyol. The hydroxyl number can also be defined by the equation:

OH = (56.1 x 1000 x f) m.w.

where

OH = hydroxyl number of the polyol

f = functionality, that is, average number of hydroxyl groups per molecule of polyol

m.w. = number average molecular weight of the polyol

Substantially any polyol used in the art to make polyurethanes can be used in making the foamable reaction mixtures of the invention. The polyols usually have an average molecular weight of greater than 4,000 and particularly at least 10,000. A particularly useful group of polyols are those marketed by Union Carbide under the trademark NIAX®, E series. The polyols preferably contain at least 40 mole percent of primary hydroxyl groups. Illustrative polyols useful in producing foamable composition used in the invention are the polyhydroxyalkanes and the polyoxyalkylene polyols.

Among the polyols which can be employed are those selected from one or more of the following classes of compositions, alone or in a mixture, known to those skilled in the polyurethane art:

(a) Alkylene oxide adducts of polyhydroxyalkanes;

(b) Alkylene oxide adducts of non-reducing sugars and sugar derivatives;

(c) Alkylene oxide adducts of phosphorus and polyphosphorus acids;

(d) Alkylene oxide adducts of polyphenols;

(e) The polyols from natural oils such as castor oil.

The polyol and polymer polyol are not to be the same as the ethylene oxide adduct of the di- or trifunctional compound. The polyols of categories (a) and (d) above do not contain only ethylene oxide as they would solids. The polyol and polymer polyol must be liquids. Such polyols have less than 25 percent of ethylene oxide and more than 75 percent of another alkylene oxide, such as, propylene oxide. The use of more than 25 weight percent of ethylene oxide imports negative properties and interfers with the humid aging properties.

Illustrative alkylene oxide adducts of polyhydroxyalkanes include the alkylene oxide adducts of ethylene glycol, propylene glycol, 1,3-dihydroxypropane, 1,3-dihydroxybutane, 1,4-dihydroxybutane, 1,4-, 1,5- and 1,6-dihydroxyhexane, 1,2-, 1,3-, 1,4-, 1,6- and 1,8-dihydroxyoctane, 1,10-dihydroxydecane, glycerol, 1,2,4-trihydroxybutane, 1,2,6-trihydroxyhexane, 1,1,1- trimethylolethane, 1,1,1-trimethylolpropane, pentaerythritol, polycaprolactone, xylitol, arabitol, sorbitol and mannitol. A preferred class of alkylene oxide adducts of polyhydroxyalkanes are the propylene oxide adducts and the propylene oxide-ethylene oxide adducts of di- and/or trihydroxyalkanes.

The alkylene oxide adducts of phosphorus and polyphosphorus acids are another useful class of polyols. Ethylene oxide, 1,2-epoxypropane, the epoxybutanes, 3-chloro- 1,2-epoxypropane, and the like are preferred alkylene oxides. Phosphoric acid, phosphorus acid, the polyphosphoric acids such as tripolyphosphoric acid and the polymetaphosphoric acids are desirable for use in this connection. A further class of polyols which can be employed are the alkylene oxide adducts of the non-reducing sugars, wherein the alkylene oxides have from 2 to 4 carbon atoms. Among the non-reducing sugars and sugar derivatives contemplated are sucrose, alkyl glycosides such as methyl glucoside, ethyl glucoside, and the like, glycol glycosides such as ethylene glycol glucoside, propylene glycol glucoside, glycerol glucoside and 1,2,6-hexanetriol glucoside, as well as the alkylene oxide adducts of the alkyl glycosides as set forth in U.S. -A-3,073,788. A still further useful class of polyols is the polyphenols, and preferably the alkylene oxide adducts thereof wherein the alkylene oxides have from 2 to 4 carbon atoms. Among the polyphenols which are contemplated are for example, bisphenol A, bisphenol F, condensation products of phenol and formaldehyde, the novolac resins; condensation products of various phenolic compounds and acrolein; the simplest member of this class being the 1,1,3-tri(hydroxyphenyl) propanes, condensation products of various phenolic compounds and glyoxal, glutaraldehyde, and other dialdehydes, the simplest members of this class being the 1,1,2,2-tetrakis-(hydroxyphenol) ethanes.

Indeed, any material having an active hydrogen as determined by the Zerewitinoff test can be utilized as the polyol also known as "polyahls". For example, amine-terminated polyether polyols are known and may be utilized, if desired.

The polyols employed can have hydroxyl numbers which vary over a wide range and are suitable to provide flexible or semi-flexible polyurethane foams from reaction with polyisocyanate. In general, the hydroxyl numbers of the polyols employed can range from 20 (and lower) to 150 (and higher). The hydroxyl number is defined as the number of milligrams of potassium hydroxide required for the complete hydrolysis of the fully acetylated or phthalated derivative prepared from 1 gram of polyols. The hydroxyl number also is defined by multiplying 56.1 times 1000 time functionality (i.e., the average number of hydroxyl groups per

molecule of polyol) and dividing the product by the molecular weight of the product. The exact polyol employed depends upon the end-use of the polyurethane product to be produced. The molecular weight or the hydroxyl number is selected properly to result in flexible or semi-flexible or rigid foams when the polyol is converted to a polyurethane. The polyols preferably possess a hydroxyl number of from 50 to 150 for semi-flexible foams, over 150 for rigid foams, and from 30 to 70 for flexible foams but can be as low as 20. Such limits are not intended to be restrictive, but are merely illustrative of the large number of possible combinations of the above polyol coreactants.

The most preferred polyols employed in the invention include the poly(oxypropylene) glycols, triols and higher functionality polyols. These polyols also include poly(oxypropylene-oxyethylene) polyols; however, desirably, the oxyethylene content should comprise less than 80 percent of the total and preferably less than 60 percent. The ethylene oxide when used can be incorporated in any fashion along the polymer chain. Stated another way, the ethylene oxide can be incorporated either in internal blocks, as terminal blocks, or may be randomly distributed along the polymer chain. Most preferably, the ethylene oxide when used is incorporated as terminal blocks, i.e., capping units. Polymer polyols can be used and are preferred as a part or all of the polyol requirements of the foamable formulation used in this invention and are also well known, the basic patents being U.S. -A-3,304,273; 3,523,093; and 3,383,351. Reissue Patent No. 28,715 (reissue of 3,383,351). Other disclosures of polymer polyols include, for example, and CA- 735,010 and 785,835; U.S. -A-3,823,201; U.S. -A- 3,953,393; U.S. -A-3,655,553; U.S.-A- 4,119,586; the PHD polyols of U.S. -A- 3,325,421; and the PIPA polyols of U.S. -A- 4,374,209. These as well as any other suitable polymer polyol can be employed herein. The polymer of the polymer polyol is formed by polymerizing one or more polymerizable ethylenically unsaturated monomers. The proportion of polymer in the polymer polyol can range from 4 to 50 weight percent, preferably from 15 to 35 weight percent, based on the total weight of the polymer polyol. The polymer is preferably formed in situ with the polyol which can be any of the polyols described above. The polymerizable ethylenically unsaturated monomers which can be used making the polymer polyols employed in this invention include the polymerizable ethylenically unsaturated hydrocarbon monomers and polymerizable ethylenically unsaturated organic monomers the molecules of which are composed of carbon, hydrogen and at least one of 0, 5, or N. These monomers are characterized by the presence therein of at least one polymerizable ethylenic unsaturated group of the type >C=C<. The monomers can be used singly or in combination to produce homopolymer polyol or copolymer polyol reactive compositions. These monomers are well-known in the art and include the hydrocarbon monomers such as styrene, alpha-methylstyrene, para-methylstyrene, 2,4-dimethylstyrene, ethylstyrene, isopropylstyrene, butylstyrene, phenylstyrene, cyclohexylstyrene and benzylstyrene; the acrylic and substituted acrylic monomers such as acrylic acid, methacrylic acid, methyl acrylate, 2-hydroxyethyl acrylate, 2-hydroxyethyl methacrylate, methyl methacrylate, octyl methacrylate, acrylonitrile, methacrylonitrile, 2-ethylhexyl acrylate, phenyl acrylate, phenyl methacrylate and N,N-dimethylacrylamide; the vinyl esters, vinyl alcohol, and vinyl ketones, such as, vinyl acetate, vinyl alcohol, vinyl butyrate, vinyl acrylate, vinyl methacrylate and N-vinyl-pyrrolidone; the vinyl halides and vinylidene halides, such as, vinyl chloride, vinyl fluoride and vinylidene chloride ; t-butylaminoethyl methacrylate, glycidyl acrylate, allyl alcohol and vinyl pyridine. Any of the known polymerizable monomers can be used and the compounds listed above are illustrative and not restrictive of the monomers suitable for use in this invention. Any of the known chain transfer agents can be present if desired. The preferred monomer used to make the polymer of the polymer polyol used in this invention is acrylonitrile alone is a homopolymer or in combination with styrene or methyl methacrylate as a copolymer. The relative weight proportions of acrylonitrile to styrene illustratively range from 20:80 to 100:0, preferably from 25:75 to 100:0 and more preferably, when low molecular weight polyols, e.g., below 2000 are used, then the weight ratio should be from 60:40 to 85:15.

Conceptually, a wide variety of monomers may be utilized in the preparation of the polymer/polyol compositions in accordance with the invention. Numerous ethylenically unsaturated monomers are disclosed in the prior patents. Any of these monomers should be suitable.

The selection of the monomer or monomers used will depend on considerations such as the relative cost of the monomers and the polyurethane product characteristics required for the intended application. To impart the desired load-bearing to the foams, the monomer or monomers used in preparing the polymer/polyol should, of course, desirably be selected to provide a polymer which has a glass transition of at least slightly hgiher than room temperature. Exemplary monomers include styrene and its derivatives such as para-methylstyrene, acrylates, methacrylates such as methyl methacrylate, acrylonitrile and other nitrile derivatives such as methacrylonitrile. Vinylidene chloride can also be employed.

The preferred monomer mixtures used to make the polymer/polyol compositions are mixtures of acrylonitrile and styrene or acrylonitrile, styrene and vinylidene chloride.

The monomer content will be typically selected to provide the desired solids content required for the

anticipated end-use application. In general, it will usually be desirable to form the polymer polyols with as high a resulting polymer or solids content as will provide the desired viscosity and stability properties.

## CATALYSTS

The catalysts that are useful in producing molded cellular high resilience flexible polyurethane in accordance with this invention include: (a) tertiary amines such as bis(dimethylaminoethyl) ether, trimethylamine, triethylamine, N-methylmorpholine, N-ethylmorpholine, N,N-dimethylbenzylamine, N,N-dimethylethanolamine, N,N,N′,N′-tetramethyl-1,3-butanediamine, triethylenediamine, triethylanolamine and pyridine oxide ; (b) tertiary phosphines such as trialkylphosphines and dialkylbenzylphosphines; (c) strong bases such as alkali and alkaline earth metal hydroxides, alkoxides, and phenoxides; (d) acidic metal salts of strong acids such as ferric chloride, stannic chloride, stannous chloride, antimony trichloride, bismuth nitrate and chloride; (e) chelates of various metals such as those which can be obtained from acetylacetone, benzoylacetone, trifluoroacetylacetone, ethyl acetoacetate, salicylaldehyde, cyclopentanone-2-carboxylate, acetyl-acetoneimine, bis-acetylacetone-alklenediimines and salicylaldehydoimine, with the various metals, such as, Be, Mg, Zn, Cd, Pb, Ti, Zr, Sn, As, Bi, Cr, Mo, Mn, Fe, Co, Ni, or such ions as $MoO^{2+}$, $UO^{2+}$, and the like; (f) alcoholates and phenolates of various metals such as $Ti(OR)_4$, $Sn(OR)_4$, $Sn(OR)_2$ and $Al(OR)_3$, wherein R is alkyl or aryl and the reaction products of alcoholates with carboxylic acids, beta-diketones, and 2-(N-N-dialkylamino) alkanols, such as the well-known chelates of titanium obtained by said or equivalent procedures; (g) salts of organic acids with a variety of metals such as alkali metals, alkaline earth metals, Al, Sn, Pb, Mn, Co, Ni, and Cu, including, for example, sodium acetate, potassium laurate, calcium hexanoate, stannous acetate, stannous octoate, stannous oleate, lead octoate, metallic driers such as manganese and lead naphthenate ; and (h) organometallic derivatives of tetravalent tin, trivalent and pentavalent As, Sb, and Bi, and metal carbonyls of iron and cobalt.

Among the organotin compounds that deserve particular mention are dialkyltin salts of carboxylic acids, e.g., dibutyltin diacetate, dibutyltin dilaurate, dibutyltin maleate, dilauryltin diacetate, dioctyltin diacetate, dibutyltin-bis(4-methylaminobenzoate) and dibutylin-bis(6-methylaminocaproate), Similarly, there may be used a trialkyltin hydroxide, dialkytin oxide, dialkyltin dialkoxide, or dialkyltin dichloride. Examples of these compounds include trimethyltin hydroxide, tributyltin hydroxide, trioctyltin hydroxide, dibutyltin oxide, dioctyltin oxide, dilauryltin oxide, dibutyltin-bis(isopropoxide), dibutyltin-bis(2-dimethylaminopentylate), dibutyltin dichloride and dioctyltin dichloride.

The tertiary amines may be used as primary catalysts for accelerating the reactive hydrogen/isocyanate reaction or as secondary catalysts in combination with one or more of the above-noted metal catalysts. Metal catalysts, or combinations of metal catalysts, may also be employed as the accelerating agents, without the use of amines. The catalysts are employed in small amounts, for example from 0.001 percent to 5 percent of each, based on the total weight of the reaction mixture.

## SURFACTANTS

It is also within the scope of the invention to employ small amounts, e.g., 0.01 percent to 5.0 percent by weight, based on the total reaction mixture, of a silicone foam stabilizer. Useful silicone surfactants included blends of one or more polyols as defined hereinabove with a silicone such as an aryl modified dimethyl silicone oil or a polyphenylethyl siloxane copolymer. Other useful silicone surfactants are the "hydrolyzable" polysiloxane-polyoxyalkylene block copolymers such as the block copolymers described in U.S. -A- 2,834,748 and 2,917,480. Another useful class of foam stabilizers are the "nonhydrolyzable polysiloxane-polyoxyalkylene block copolymers such as the block copolymers described in U.S. -A- 3,505,377; and GB-1,220,471. The latter class of copolymers differs from the above-mentioned polysiloxane-polyoxyalkylene block copolymers in that the polysiloxane moiety is bonded to polyoxyalkylene moiety through carbon-to-oxygen-to-silicon bonds. These various polysiloxane-polyoxyalkylene block copolymers preferably contain from 5 to 50 weight percent of polysiloxane polymer with the remainder being polyoxyalkylene polymer. Any other suitable surfactants or stabilizers can be used.

High molecular weight, high resilience polyurethane foams need a foam stabilizer (in addition to the silicone surfactant).

## PIGMENTS

Any compatible pigment can be used in the foamable mixture used in this invention. Carbon black is extensively used as a pigment in polyurethane products. Other useful pigments include Prussian blue, manganese violet, manganese blue, emerald green, cobalt blue, cobalt violet, Mayan blue, iron oxide red, chrome red, vermillion, ultramarine blue, ultramarine violet, phthalocyanine green and brilliant red. The amounts of pigments used are not narrowly critical and depend in large part on the shade of medium to dark color desired. Illustrative ranges are from 0.1 to 4 weight percent, preferably 0.3 to 2 weight percent of the pigment based on the weight of the polyurethane product. Larger amounts of pigment can be used although difficulties with mixing and handling the larger amounts of pigments can result because of increased viscosity. The pigment can be incorporated into the polyol, the polyisocyanate composition, or both but preferably is added to the preblend.

## BLOWING AGENTS

A small amount of a polyurethane blowing agent, such as, water, is used in the foamable reaction mixture (for example, from 0.1 to 5 weight percent of water or more, based upon total weight of the polyol composition), or through the use of blowing agents which are vaporized by the exotherm of the reaction, or by a combination of the two methods. Illustrative polyurethane blowing agents include: the halogenated hydrocarbons such as trichloromonofluoromethane, dichlorodifluoromethane, dichloromonofluoromethane, dichloromethane, trichloromethane, 1,1-dichloro-1-fluoroethane, 1,1,2-trichloro-1,2,2-trifluoroethane, hexafluorocyclobutane and octafluorocyclobutane; water and carbon dioxide gas or a compound which generates carbon dioxide; the lower molecular weight alkanes and alkenes; the lower weight dialkyl ethers, e.g., acetone; and mixtures thereof. Another class of blowing agents include thermally unstable compounds which liberate gases upon heating, such as N,N′-dimethyl-N,N′-dinitrosoterephthalamide. The quantity of blowing agent employed will vary with factors, such as, the density desired in the foamed product, but often 1 to 50 weight percent, based on the total weight of the polymer/polyol, of the non-water blowing agent is used.

A lowering of the water level generally causes an increase in the foam density and a reduction in the wet set value.

## ISOCYANATES

The organic polyisocyanates that are useful in producing the molded flexible polyurethane cellular products in accordance with this invention are organic compounds that contain at least two isocyanate groups and include the monomeric and polymeric organic polyisocyanates such as prepolymers produced by reacting a polyol with an excess of a polyisocyanate. The quasi-prepolymers such as the reaction products of excess tolylene diisocyanate and short chain polyoxypropylene diols or triols, are preferred in those instances where ease of processing such materials is desired. The polyisocyanates are well-known in the art. Suitable organic polyisocyanates include the hydrocarbon diisocyanates (e.g., the alkylene diisocyanates and the arylene diisocyanates) as well as triisocyanates. Examples of suitable polyisocyanates are 1,8-diisocyanatooctane, 1,5-diisocyanato-2,2,4-trimethylpentane, 1,9-diisocyanatononane, 1,1 -diisocyanatopcopyl ether of 1,4-butylene glycol, 1,11-diisocyanatoundecane, 1,12-diisocyanatododecane, bis(isocyanatohexyl) sulfide, 1,4- diisocyanatobenzene, 2,4-diisocyanatotoluene, 2,6-diisocyanatotoluene and mixtures thereof, 1,3-diisocyanato-o-xylene, 1,3-diisocyanato-m-xylene, 1,3-diisocyanato-p-2,4-diisocyanato-1-chlorobenzene, 2,4-diisocyanato-1-nitrobenzene, and 2,5-diisocyanato-1-nitrobenzene, 4,4′-diphenylmethylene diisocyanate, 3,3′-diphenylmethylene diisocyanate, 2, 4′-diphenylmethylene diisocyanate, modified diphenylmethylene diisocyanates modified with carbodiimides to liquify same, and poly-methylene poly(phenyleneisocyanates) having the formula:

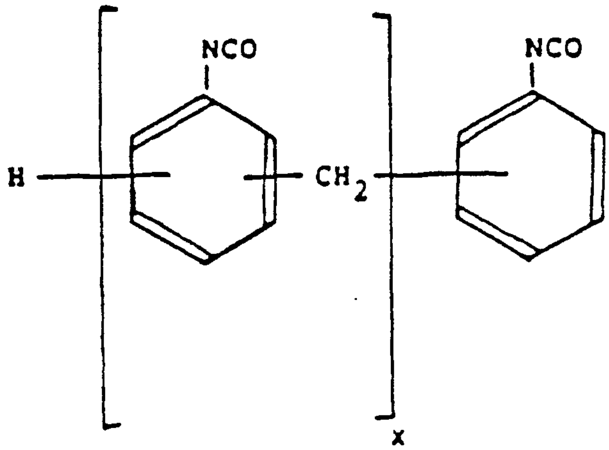

wherein x has an average value from 1.1 to 5 inclusive (preferably from 2.0 to 3.0), and mixtures thereof. A useful mixture of polyisocyanates comprises 80 weight percent 2,4-tolylenediisocyanate and 20 weight percent 2,6-tolylenediisocyanate.

Using the polyol component as the base ( i.e., 100 percent), the isocyanate compound is often used in an amount ranging from an index of 95 to 110, which is 40.4 to 46.8 parts by weight, respectively, when 100 parts by weight is the total of the polyol and isocyanate compounds. The compound preferably has an index of about 105, which is about a 5 percent excess.

Broadly, the organic polyisocyanates which can be used in the preparation of foams can be represented by the following formula:

$R(NCO)_z$

wherein R is a polyvalent organic radical which is either aliphatic, aralkyl, alkaryl, aromatic or mixtures thereof, and z is an integer which corresponds to the valence of R and is at least two.

In general, a lowering of the catalyst amount improves the wet set value but the cure rate is often adversely effected.

The polyurethane formulations can also optionally contain auxiliary agents and additives, such as, phenolic antioxidants, flame-retarding agents, fungistatic substances, bacteriostatic substances, plasticizers and pore regulators.

## CHAIN EXTENDERS/STABILIZERS

Under certain circumstances, it may be advantageous to use chain extenders for the preparation of the polyurethane foams. Representative examples of such materials include di- and trifunctional compounds having molecular weights of 17 to 300. The following are specific examples of such compounds: di- and trialkanolamines such as diethanolamine and triethanolamine, aliphatic and aromatic diamines such as ethylenediamine, 1,4-butanediamine, 1,6-hexamethylenediamine, 4,4′-diaminodiphenylmethane, 2,4- and 2,6-toluenediamine, and preferably the aliphatic diols and triols having 2 to 6 carbon atoms such as ethylene glycol, 1,4-butanediol, 1,6-hexamethylene glycol, glycerine, and trimethylol propane. If chain extenders are used, they are used in quantities of 1 part to 60 parts, preferably 10 to 30 parts by weight per 100 part by weight of polyol.

## PLASTICIZERS/FLAME RETARDANTS

It may also be advantageous to include a plasticizer in the reaction mixture in order to reduce brittleness of the foams. Commonly known plasticizers may be used. It is particularly advantageous to use those materials which contain phosphorus and/or halogen atoms and which, therefore, additionally increase the flame resistance of the polyurethane plastics. These materials include tricresyl phosphate, tris-2-chloroethyl phosphate, trischloropropyl phosphate, and tris-2,3-dibromopropyl phosphate.

In addition to the already mentioned halogen-substituted phosphates, inorganic flame retardants may also be used to render the polyurethane foams flame resistant. Examples of these include antimony trioxide, arsenic oxide, ammonium phosphate and calcium sulfate and melamine. It generally has proven to

be advantageous to use 5 to 50 parts by weight, preferably 5 to 25 parts by weight, of the referenced flame reteradants per 100 parts by weight of the polyol.

The mixed ingredients are in liquid form, such as, solutions, suspensions, dispersions and the like. Solid minor components can be dissolved, suspended, dispersed or the like in one or more of the major liquid components.

The high resilience polyurethane foams of the invention are broadly prepared by reacting a polyol component with an isocyanate component in the presence of a blowing agent and a reaction catalyst. A foam stabilizer is optionally present. The ethylene oxide adduct of the di- or trifunctional starter is present in the liquid reaction mixture. The reaction and the foam formation generally occurs at a temperature of 30° to 90°C.

To repeat, the high resilience polyurethane foam can be produced by reacting the polyol component and the ethylene oxide adduct of the di- or trifunctional starter compound with the isocyanate component as fundamental starting material in the presence of a blowing agent, a reaction catalyst, and a foam stabilizer. The blends can be formed into a variety of foamed shapes using known and conventional mold techniques.

The polyurethane foams can be produced according to the prepolymer or preferably according to the one-shot process. If the prepolymer process is used, it is advantageous to react small quantities of the polymer polyol, di-trifunctional starter compound and organic polyisocyanate in a prior reaction stage to form a urea and possibly urethane group containing polyisocyanate. According to the one-shot process, the starting components, auxiliaries and additives are introduced individually via several feed nozzles when one mixing chamber is used and are intensively mixed in the mixing chamber. However, it may be advantageous to work according to the two-component process and to combine the polyols, catalysts, blowing agents and optionally the chain extenders or cross-linking agents, auxiliaries and additives in the so-called A component and to use the organic polyisocyanates, optionally as a mixture with physical blowing agents auxiliaries and additives as the B component. The A and B components are then intensively mixed prior to manufacture of the polyurethane foams. The most preferred preparation method is set out in Example 1.

Foams can be produced by either the stabstock (free-rise) or molding process. In the slabstock process, the urethane foam ingredients are mixed and then deposited onto a moving conveyor belt which has been covered with a release paper or film. After the foam has risen (completed expansion) and cured sufficiently the release paper or film is removed and the foam is cut into blocks for further fabrication.

A detailed description of the molding process is given below.

In the preferred method for molding the ingredient mixtures are preheated to 21 - 27°C (70° to 80°F), best about 75°F, before being placed in the mold. The reaction is preferably conducted at 60°C (140°) to 68°C, (155°F) best about 65°C (150°F), so the mold is heated to that temperature before the preheated ingredient mixture is inserted therein and is maintained at that temperature until the foamed polyurethane is formed.

The high resilience polyurethane foam moldings can be made using any suitable mold. The molds disclosed in commonly-owned U.S. Patent Application Serial No. 221,758, (D-15391), filed on July 20, 1988, applicant: Keith Douglas Cavender, title: "Improved Mold And Mold Vent", are particularly useful.

The preferred method for producing the foamable molded product is: a preheated mixture of the ingredients that are to be expanded to form the foamed product are placed into a mold having an internal cavity which defines the exterior shape of the desired molded product. The mold is then closed. The foaming reaction begins when the mixture of ingredients is formed and continues after the mold is closed. As the foaming mixture expands to fill the mold cavity it must force out the air that is within the cavity. If this air is not removed from the mold the mold is not completely filled with foaming mixture and the end foamed product would have a considerable number of voids. The technique that is used to remove this air consists of one or more air vents. Although the mold cavity must be fully filled with the foaming mixture, the foaming mixture should not exude to any great extent out of the mold cavity through one or more of the air vent openings.

The foaming can be conducted at a relatively low temperature of below 80° to 90°C.

In commercial plants, the molding operations often consist of apparatus equipped with multiple mold carriers (each of which may have more than one mold). The molds are fed by a foam machine having one or more high pressure (mix) heads.

## GLOSSARY OF TERMS

Polyol A - A polyol made by reacting propylene oxide with glycerine in the presence of potassium

hydroxide catalyst. capping with ethylne oxide and refining to remove catalyst. The polyol contains about 13 weight percent of ethylene oxide as a cap and has a hydroxyl number of about 28.

Polyol B - Same as Polyol A. but the polyol contains about 16.5 weight percent of ethylene oxide as a cap and has a hydroxyl number of about 28.

Polyol C - Same as Polyol A. but the polyol contains about 16.5 weight percent of ethylene oxide as a cap and has a hydroxyl number of about 35.

Polyol D - Same as Polyol A. but glycerine is replaced with sorbitol and the polyol contains about 16 weight percent of ethylene oxide as a cap and has a hydroxyl number of about 28.

Polyol E - Same as Polyol A. but the polyol contains about 19 weight percent of ethylene oxide as a cap and has a hydroxyl number of about 35.

Polyol F - Same as Polyol A. but glycerine is replaced with pentaerythritol and the polyol contains about 15 weight percent of ethylene oxide as a cap and has a hydroxyl number of about 28.

Polyol G - Same as Polyol F, but the hydroxyl number is about 33.

Polymer Polyol A - A polymer polyol sold by Mitsui Toatsu, Inc. as "POP-3218". It contains 20 weight percent of polymer and has a hydroxyl number of 28.

Polymer Polyol B - A polymer polyol sold by Union Carbide Corporation as "Max Polyol E-515". It contains 28 weight percent of polymer and has a hydroxyl number of about 20.

Polymer Polyol C - A polymer polyol sold by Union Carbide Corporation as "NIAX Polyol E-519". It contains 28 weight percent of polymer and has a hydroxyl number of about 25.

Polymer Polyol D - A polymer polyol sold by Union Carbide Corporation as "NIAX Polyol E-585". It contains 28 weight percent of polymer and has a hydroxyl number of about 20.

Polymer Polyol E - A polymer polyol sold by Union Carbide Corporation as "NIAX Polymer E-688". It contains 28 weight percent of polymer and has a hydroxyl number of about 22.

Additive A - An ethylene oxide adduct of glycerine to a number average molecular weight of about 1.000.

Additive B - An ethylene oxide adduct of glycerine to a number average molecular weight of about 2,000.

Catalyst A - A polyurethane foam bis(2-dimethyl-aminoethyl)ether catalyst sold as "NIAX® Catalyst A-1" by Union Carbide Corporation.

Catalyst B - A polyurethane foam bis(2-dimethylaminoethyl)ether catalyst sold as "NIAX® Catalyst A-107" by Union Carbide Corporation.

Catalyst C - A polyurethane foam triethylenediamine catalyst sold as "NIAX® Catalyst A-33" by Union Carbide Corporation.

Catalyst D - A polyurethane foam bis(2-dimethylaminoethyl)ether and triethylene-diamine catalyst sold as "NIAX® Catalyst C-225" by Union Carbide Corporation.

Catalyst E - A polyurethane foam triethylenediamine catalyst sold as "NIAX®" Catalyst C-229" by Union Carbide Corporation.

Surfactant A - A silicone surfactant sold for use in high resiliency foam by Union Carbide Corporation as "Silicone Surfactant Y-10,575".

Surfactant B - Same as Surfactant A. but sold as "Silicone Surfactant L-5309".

DEOA - Diethanolamine.

KL-200 - An ethylene oxide adduct of pentaerythritol produced by Mitsui Toatsu Chemicals, Inc.

KL-210 - A mixture of KL-200 and diethanolamine produced by Mitsui Toatsu Chemicals, Inc.

PC-77 - A polyurethane foam pentamethyldipropylenetriamine catalyst sold by Air Products Co.

TDI is a mixture of 80 percent of 2,4-tolylenediisocyanate and 20 percent of 2,6-tolylenediisocyanate.

Rubinate M is a polymethylene polyphenyl isocyanate (MDI) manufactured by Rubicon Chemical Co.

Wet Set is a foam test in which a specimen of 50 mm x 50 mm is compressed to 15 mm (50 percent) in thickness for 22 hours at 50° C and 95 percent relative humidity (R.H.). Then the foam is released from compression for 30 minutes at 21° C (70° F) and 50 percent relatively humidity (R.H.) and the loss of thickness is measured and expressed as percent of original thickness.

Elongation is the maximum tensile extensibility expressed as percent of original length of a foam tensile specimen.

The molecular weights herein are average molecular weights, unless otherwise stated herein or otherwise obvious herefrom.

The average molecular weights for the wet set additive compositions, polyols and polymer/polyol compositions are determined by first measuring the hydroxyl number. Molecular weight is then determined as follows: The hydroxyl number is defined as the number of milligrams or potassium hydroxide required for the complete hydrolysis of the fully phthalated derivative prepared from one gram of polyol. The

hydroxyl number can also be defined by the equation:

OH = (56.1 x 1000 x f)/m.w.

wherein

OH = hydroxyl number of the polyol

f = functionality, that is, average number of hydroxyl groups per molecular of polyol

m.w. = number average molecular weight of the polyol.

IFD means Indentation Force Deflection and herein follows the ASTM test for firmness.

Herein all percentages, proportions, parts and ratios are on a weight basis and all temperatures are in °C, unless otherwise stated herein or otherwise obvious herefrom to one skilled in the art.

Example 1

A foam formulation is prepared from the ingredients listed in Table I below. The formulation is prepared by first mixing all of the ingredients except the TDI at 4000 rpm for 55 s. The mixing is done under heat so that the mixture becomes and remains about 24 °C (75 °F). (The foam chemicals are mixed in a 1 2 gallon paper carton fitted with baffles. A drill press operated turbine mixer is used for mixing.) After the mixing is stopped, the TDI is added quickly and mixing is continued for 4 moe seconds. After which the mixture is quickly poured into a cast aluminum mold (FBH type) having dimensions of 381•381•381 mm (15" x 15" x 15") and provided with a lid hinged to the mold and four small vent holes 1.6 mm (1 16 in.) provided close to each corner of the lid. The lid is provided with a latch for holding it in closed position. After pouring the resultant mixture into the mold, the lid is closed and latched. The mold temperature is about 65 °C (150 °F) - a forced-draft hot air oven was used for this purpose. The mixture in the mold is allowed to foam and rise and it fills the mold. Some of the foam extrudes through the four vent holes. The foam is allowed to set for 3 to 4 min from the pour and then the foam is demolded. The test blocks are roller crushed three times to remove all closed cells prior to testing. All foamed blocks are aged at ambient laboratory conditions for at least 3 days before testing begins. The molded foam product is tested for the items set out in Table I. In the examples: foam physical properties are determined by ASTM test methods and Japanese Wet Set test methods; and the ASTM tests are conducted according to ANSI/ASTM D3574-77 methods. A forced draft humidity chamber is employed in testing Wet Set properties. This chamber is maintained at 50 °C and 95 R.H. All tests except for Wet Set are done in a 21 ± 1 °C (70 ± 2 °F)/50 ± 2% R.H. environment.

Table I

| Components | Parts by Weight |
|---|---|
| Base Polyol E | 45 |
| Polymer Polyol E | 55 |
| Additive A | 4.0 |
| $H_2O$ | 3.3 |
| DEOA | 1.5 |
| Catalyst D | 0.40 |
| PC-77 | 0.15 |
| Surfactant A | 1.0 |
| TDI (105 index) | 42.0 |
| Molded Foam Properties | |
| Density,(pcf) kg/m$^3$ | 43.2 (2.7) |
| Resiliency, percent | 65 |
| IFD,(lbs. 50 in.$^2$) kg/cm$^2$ | |
| 25 percent | 4.2 (60) |
| 50 percent | 7.2 (110) |
| Tensile Strength, (psi) N/cm$^2$ | 19.3(28.0) |
| Elongation, percent | 105 |
| 50 percent compression set, percent $C_T$ | 3.5 |
| Wet Set, percent | 13.5 |

Comparative Example 1

A foam formulation is prepared from the ingredients listed in Table II below. The formulation is processed as in Example 1 to form a molded polyurethane product. The molded product is tested for the items set out in Table II.

Table II

| Formulation | |
|---|---|
| Components | Parts by Weight |
| Polyol E | 30 |
| Polymer Polyol E | 65 |
| KL-200 | 5 |
| $H_2O$ | 3.3 |
| DEOA | 1.0 |
| Catalyst D | 0.5 |
| Surfactant A | 1.0 |
| TDI (105 index) | 44.6 |
| Molded Foam Properties | |
| Density,(pcf) kg m$^3$ | 43.2 ( 2.7 ) |
| Resiliency, percent | 63 |
| IFD,(lbs. 50 in.$^2$) kg cm$^2$ | |
| 25 percent | 4.228 (60.4 ) |
| 50 percent | 8.015 (114.5 ) |
| Tensile Strength,(psi) N/cm$^2$ | 23.2 (33.7 ) |
| Elongation, percent | 69 |
| 50 percent compression set, percent $C_T$ | 4.2 |
| Wet Set, percent | 17.2 |

## Example 2

The procedure of Example 1 is repeated using the ingredients set out in Table III below. The polyols used were varied. The two runs, which incorporated Wet Set Additive-B provided better indentation force deflection and 50% compression set results than the other two runs.

TABLE III

| Cushion Foams | | | | |
|---|---|---|---|---|
| Polyol Effects | | | | |
| Formulation | | | | |
| Components | Parts by Weight | | | |
| Polyol-B | 60 | - | 30 | - |
| Polyol-E | - | 60 | - | 30 |
| Polymer Polyol-E | 40 | 40 | 70 | 70 |
| Additive-B | - | - | 2.0 | 2.0 |
| $H_2O$ | 3.3 | 3.3 | 3.3 | 3.3 |
| DEOA | 2.0 | 2.0 | 1.3 | 1.3 |
| Catalyst-D | 0.6 | 0.6 | 0.5 | 0.5 |
| Surfactant-A | 2.0 | 2.0 | 2.0 | 2.0 |
| TDI (105 Index) | 42.9 | 42.3 | 40.7 | 40.1 |
| Foam Properties | | | | |
| Density,(pcf) kg/$m^3$ | 46.4 (2.9) | 44.8 (2.8) | 46.4 (2.9) | 44.8 (2.8) |
| Resiliency % | 52 | 64 | 54 | 46 |
| IFD,(lbs. 50 in. 2) kg/$cm^2$ | | | | |
| 25% | 3.647 (52.1) | 3.458 ( 49.4) | 4.27 (61.0) | 4.809 (68.7) |
| 50% | 6.552 (93.6) | 6.272 (89.6) | 8.008 (114.4) | 8.981 (128.3) |
| Tensile Strength, (psi) N/$cm^2$ | 18.5 (26.8) | 21.1 (30.6) | 20.6 (29.9) | 23.5 (34.1) |
| Elongation, % | 87 | 97 | 76 | 87 |
| 50% Compression Set, % $C_T$ | 19.0 | 21.0 | 14.7 | 17.8 |

Example 3

The procedure of Example 1 is repeated using the ingredients (including Wet Set Additive-B) set out in Table IV below. This example illustrates the variation of the foam properties with the variation of the diethanolamine. An increase in the amount of DEOA lowered the wet set value, but also reduced the load bearing and elongation values. The Additive-B effectively improved the foam wet set values.

TABLE IV

Cushion Foams
DEOA Effects

| Formulation Components | Parts by Weight | | |
|---|---|---|---|
| Polyol-B | 30 | 30 | 30 |
| Polymer Polyol-E | 70 | 70 | 70 |
| Additive-B | 2 | 2 | 2 |
| H2O | 3.3 | 3.3 | 3.3 |
| DEOA | 1.0 | 1.5 | 2.0 |
| Catalyst-D | 0.5 | 0.5 | 0.5 |
| Surfactant-A | 2.0 | 2.0 | 2.0 |
| TDI (105 Index) | 40.0 | 41.3 | 42.6 |
| **Foam Properties** | | | |
| Density, (pcf) $kg/m^3$ | 46.4 (2.9) | 46.4 (2.9) | 46.4 (2.9) |
| Resiliency % | 58 | 59 | 62 |
| IFD, $kg/cm^2$ (lbs./50 $in.^2$) 25% | 5.061 (72.3) | 4.76 (68.0) | 4.151 (59.3) |
| 50% | 9.191 (131.3) | 8.596 (122.8) | 7.651 (109.3) |
| Tensile Strength, $N/cm^2$ (psi) | 21.4 (31.0) | 19.5 (28.2) | 18.9 (27.4) |
| Elongation, % | 81 | 68 | 66 |
| 50% Compression Set, % $C_T$ | 4.7 | 4.2 | 3.7 |
| Wet Set, % | 17.3 | 16.1 | 13.9 |

Example 4

The procedure of Example 1 is repeated using the ingredients set out in Table V below. This example shows that Wet Set Additive-B, compared to KL-200, is an equally effective improver of the foam wet set properties. The first three formulations show that 2 phr of Additive-B gives about the same improvement in

the wet set (-30% reduction in values) as 5 phr of KL-200. The Additive-B shows one advantage over KL-200 in that it does not reduce foam elongation nearly as much while yielding wet set improvements. The second three formulations show the effects of Additive-B level on foam wet set properties. The formulation with 1.0 phr of Additive-B appears to be anamalous in that load values are quite high and elongation is low, suggesting that the TDI is somewhat higher than it should be. Otherwise, the data shows improvement in the wet set as the Additive-B level is increased.

TABLE V

| Cushion Foams | | | | | | |
|---|---|---|---|---|---|---|
| Adduct Effects | | | | | | |
| Formulation | | | | | | |
| Components | Parts by Weight | | | | | |
| Polyol-B | - | - | - | 30 | 30 | 30 |
| Polyol-E | 35 | 30 | 35 | - | - | - |
| Polymer-Polyol-E | 65 | 65 | 65 | 70 | 70 | |
| KL-200 | - | 5 | - | - | - | - |
| Additive-B | - | - | 2 | 1.0 | 2.0 | 3.0 |
| $H_2O$ | 3.3 | 3.3 | 3.3 | 3.3 | 3.3 | 3.3 |
| DEOA | 1.0 | 1.0 | 1.0 | 1.3 | 1.3 | 1.3 |
| Catalyst-D | 0.5 | 0.5 | 0.5 | 0.5 | 0.5 | 0.5 |
| Surfactant-A | 1.0 | 1.0 | 1.0 | 2.0 | 2.0 | 2.0 |
| TDI (105 Index) | 40.6 | 44.6 | 40.6 | 40.7 | 40.7 | 40.7 |
| Foam Properties | | | | | | |
| | 44.8 | 43.2 | 44.8 | 46.4 | 46.4 | 46.4 |
| Density, (pcf) kg/m$^3$ | (2.8) | (2.7) | (2.8) | (2.9) | (2.9) | (2.9) |
| Resiliency % | 43 | 63 | 43 | 50 | 53 | 58 |
| kg/cm$^2$ | | | | | | |
| IFD,(lbs./50 in.2) | 4.627 | 4.228 | 4.228 | 5.565 | 4.501 | 4.256 |
| 25% | (66.1) | (60.4) | (60.4) | (79.5) | (64.3) | (60.8) |
| | 8.491 | 8.015 | 8.001 | 9.87 | 8.281 | 7.665 |
| 50% | (121.3) | (114.5) | (114.3) | (141.0) | (118.3) | (109.5) |
| N/cm$^2$ | 23.3 | 23.2 | 20.7 | 22.7 | 20.7 | 17.5 |
| Tensile Strength, (psi) | (33.8) | (33.7) | (30.0) | (32.9) | (30.0) | (25.4) |
| Elongation, % | 105 | 69 | 91 | 78 | 78 | 76 |
| 50% Compression Set, % $C_T$ | 5.4 | 4.2 | 4.4 | 4.7 | 4.6 | 4.1 |
| Wet Set, % | 24.5 | 17.2 | 17.3 | 20.4 | 16.3 | 13.6 |

## Example 5

The procedure of Example 1 is repeated using the ingredients (including Additive-B or Additive-A) set out in Table VI below. This example shows the effect of both Additives on foam properties made with Polyol D and Polyol E. Increasing the concentration of each one is observed to reduce the foam wet set values significantly. While Additive-A is seen to be about half as effective as an equal amount of Additive-B the Additive-A is preferred because it has less adverse effect on foam stability. Also, Additive-A is a liquid at temperatures around 25°C and, therefore, easier to use. Additive-A appears to offer a slight advantage in foam elongation properties.

TABLE VI

| Cushion Foam | | | | | | |
|---|---|---|---|---|---|---|
| Adduct Effects | | | | | | |
| Formulations | | | | | | |
| Components | Parts by Weight | | | | | |
| Polyol-D | 40 | 40 | 40 | 40 | 40 | 40 |
| Polymer Polyol-E | 60 | 60 | 60 | 60 | 60 | 60 |
| $H_2O$ | 3.3 | 3.3 | 3.3 | 3.3 | 3.3 | 3.3 |
| DEOA | 1.5 | 1.5 | 1.5 | 1.5 | 1.5 | 1.5 |
| Additive-B | 2.0 | 3.0 | 4.0 | - | - | - |
| Additive-A | - | - | - | 2.0 | 3.0 | 4.0 |
| Catalyst-D | 0.55 | 0.55 | 0.55 | 0.55 | 0.55 | 0.55 |
| Surfactant-A | 2.0 | 2.0 | 2.0 | 2.0 | 2.0 | 2.0 |
| TDI (105 Index) | 41.7 | 41.7 | 41.7 | 41.7 | 41.7 | 41.7 |
| Molded Foam Properties | | | | | | |
| Density, (pcf) kg/m$^3$ | 44.8 (2.8) | 44.8 (2.8) | 44.8 (2.8) | 44.8 (2.8) | 44.8 (2.8) | 44.8 (2.8) |
| Resiliency % | 65 | 68 | 65 | 65 | 65 | 65 |
| IFD,(lbs. 50 in.)kg/cm$^2$ 25% | 4.543 (64.9) | 4.368 (62.4) | 4.032 (57.6) | 4.34 (62.0) | 4.172 (59.6) | 4.445 (63.5) |
| 50% | 8.26 (118.0) | 7.966 (113.8) | 7.399 (105.7) | 7.791 (111.3) | 7.581 (108.3) | 8.085 (115.5) |
| N/cm$^2$ Tensile Strength, (psi) | 16.8 (24.3) | 15.7 (22.7) | 16.0 (23.2) | 20.7 (30.0) | 17.9 (26.0) | 18.1 (26.3) |
| Elongation, % | 78 | 76 | 68 | 91 | 88 | 83 |
| 50% Compression Set, % $C_T$ | 3.9 | 3.6 | 3.7 | 4.4 | 4.0 | 3.7 |
| Wet Set, % | 12.5 | 11.0 | 9.8 | 16.0 | 14.2 | 12.9 |

## Example 6

The procedure of Example 1 is repeated using the ingredients (including Additive-B and Additive-A) set out in Table VII below. This example further illustrates the results discussed in Example 5.

TABLE VII
Cushion Foam
Adduct Effects

Formulations

| Components | Parts by Weight | | | | | | | |
|---|---|---|---|---|---|---|---|---|
| Polyol-E | 35 | 35 | 35 | 35 | 40 | 40 | 40 | 40 |
| Polymer Polyol-E | 65 | 65 | 65 | 65 | 60 | 60 | 60 | 60 |
| Additive-B | - | 1.0 | 2.0 | 3.0 | - | - | - | - |
| Additive-A | - | - | - | - | 2.0 | 3.0 | 4.0 | 5.0 |
| $H_2O$ | 3.3 | 3.3 | 3.3 | 3.3 | 3.3 | 3.3 | 3.3 | 3.3 |
| DEOA | 1.0 | 1.0 | 1.0 | 1.0 | 1.0 | 1.0 | 1.0 | 1.0 |
| Catalyst-D | 0.5 | 0.5 | 0.5 | 0.5 | 0.5 | 0.5 | 0.5 | 0.5 |
| Surfactant-A | 1.0 | 1.0 | 1.0 | 1.0 | 1.0 | 1.0 | 1.0 | 1.0 |
| TDI (105 Index) | 40.0 | 40.0 | 40.0 | 40.0 | 41.8 | 41.8 | 41.8 | 40.8 |

Foam Properties

| | | | | | | | | |
|---|---|---|---|---|---|---|---|---|
| Density, (pcf) $kg/m^3$ | 43.2 (2.7) | 43.2 (2.7) | 43.2 (2.7) | 43.2 (2.7) | 43.2 (2.7) | 43.2 (2.7) | 43.2 (2.7) | 43.2 (2.7) |
| Resiliency % | 52 | 52 | 58 | 56 | 62 | 63 | 65 | 64 |
| IFD, $kg/cm^2$ (lbs./50 in.) 25% | 5.866 (83.8) | 5.621 (80.3) | 5.481 (78.3) | 4.886 (69.8) | 4.368 (62.4) | 4.228 (60.4) | 4.191 (59.3) | 4.102 (58.6) |
| 50% | 10.486 (149.8) | 10.101 (144.3) | 9.681 (138.3) | 8.68 (124.0) | 7.798 (111.4) | 7.462 (106.6) | 7.371 (105.3) | 7.273 (103.9) |
| Tensile Strength, (psi) $N/cm^2$ | 23.2 (33.6) | 21.8 (31.6) | 23.2 (33.6) | 19.8 (28.7) | 24.8 (36.0) | 19.3 (28.0) | 19.3 (28.0) | 17.9 (26.0) |
| Elongation, % | 98 | 94 | 95 | 84 | 104 | 94 | 93 | 84 |
| 50% Compression Set, $\%C_T$ | 5.1 | 4.7 | 4.0 | 4.1 | 3.5 | 3.5 | 3.5 | 3.1 |
| Wet Set, % | 23.2 | 19.3 | 15.8 | 14.9 | 15.8 | 14.1 | 12.8 | 10.9 |

## Example 7

The procedure of Example 1 is repeated using the ingredients (including Additive-B) set out in Table VIII below. This example illustrates the variation of the foam properties with the variation of the added diethanolamine.

TABLE VIII

| Cushion Foam | | | | | | |
|---|---|---|---|---|---|---|
| DEOA Effects | | | | | | |
| Formulations | | | | | | |
| Components | Parts by Weight | | | | | |
| Polyol-E | 45 | 45 | 45 | 45 | 45 | 45 |
| Polymer Polyol-E | 55 | 55 | 55 | 55 | 55 | 55 |
| Additive-B | 2.0 | 2.0 | 2.0 | 2.0 | 2.0 | 2.0 |
| $H_2O$ | 3.3 | 3.3 | 3.3 | 3.3 | 3.3 | 3.3 |
| DEOA | 1.0 | 1.3 | 1.5 | 1.8 | 2.0 | 2.5 |
| Catalyst-D | 0.4 | 0.4 | 0.4 | 0.4 | 0.4 | 0.4 |
| Surfactant-A | 1.0 | 1.0 | 1.0 | 1.0 | 1.0 | 1.0 |
| TDI (105 Index) | 40.6 | 41.5 | 42.0 | 42.7 | 43.3 | 44.6 |
| Molded Foam Properties | | | | | | |
| Density, (pcf) kg/$m^3$ | 43.2 (2.7) | 43.2 (2.7) | 43.2 (2.7) | 43.2 (2.7) | 43.2 (2.7) | 43.2 (2.7) |
| Resiliency % | 65 | 65 | 66 | 67 | 68 | 69 |
| kg/$cm^2$ | | | | | | |
| IFD,(lbs. 50 in.$^2$) 25% | 4.921 (70.3) | 4.865 (69.5) | 4.466 (63.8) | 4.76 (68.0) | 4.501 (64.3) | 4.361 (62.3) |
| 50% | 8.526 (121.8) | 8.421 (120.3) | 7.84 (112.0) | 8.26 (118.0) | 9.945 (113.5) | 7.686 (109.8) |
| Tensile Strength, (psi) N/$cm^2$ | 20.1 (29.1) | 18.1 (26.3) | 18.9 (27.4) | 20.8 (30.1) | 18.8 (27.2) | 18.8 (27.2) |
| Elongation, % | 97 | 96 | 95 | 91 | 91 | 89 |
| 50% Compression Set, $\%C_T$ | 3.7 | 3.4 | 3.5 | 3.3 | 3.4 | 3.1 |
| Wet Set, % | 13.6 | 11.9 | 12.2 | 11.7 | 12.5 | 12.6 |

## Example 8

The procedure of Example 1 was repeated using the ingredients (including Additive-B) set out in Table IX below. This example illustrates the variation of the foam properties with the variation of isocyanate catalyst. The data of Table IX shows that increasing TDI Index from 100 to 111 increases foam loads, reduces elongations, and reduces wet set values to a minor extent. Apparently, TDI index levels above 100 have much less effect on foam wet set values than TDI Index levels below 100. The limited experimental data suggests that foam wet set properties deteriorate rapidly as the TDI Index is reduced below 95.

TABLE IX

| Cushion Foam | | | | | | |
|---|---|---|---|---|---|---|
| TDI Index Effects | | | | | | |
| Formulations | | | | | | |
| Components | Parts by Weight | | | | | |
| Polyol-E | 35 | 35 | 35 | 35 | 35 | 35 |
| Polymer Polyol-E | 65 | 65 | 65 | 65 | 65 | 65 |
| Additive-B | 2.0 | 2.0 | 2.0 | 2.0 | 2.0 | 2.0 |
| $H_2O$ | 3.3 | 3.3 | 3.3 | 3.3 | 3.3 | 3.3 |
| DEOA | 1.0 | 1.0 | 1.0 | 1.0 | 1.0 | 1.0 |
| Catalyst-D | 0.5 | 0.5 | 0.5 | 0.5 | 0.5 | 0.5 |
| Surfactant-A | 1.0 | 1.0 | 1.0 | 1.0 | 1.0 | 1.0 |
| TDI | 38.7 | 39.5 | 40.6 | 41.4 | 42.2 | 43.0 |
| TDI Index | 100 | 102 | 105 | 107 | 109 | 111 |
| Molded Foam Properties | | | | | | |
| Density, (pcf) kg/$m^3$ | 43.2 (2.7) | 43.2 (2.7) | 43.2 (2.7) | 43.2 (2.7) | 43.2 (2.7) | 43.2 (2.7) |
| Resiliency % | 61 | 59 | 60 | 59 | 58 | 61 |
| kg/$cm^2$ | | | | | | |
| IFD,(lbs./50 in.) 25% | 5.033 (71.9) | 4.858 (69.4) | 5.04 (72.0) | 5.257 (75.1) | 5.166 (73.8) | 5.306 (75.8) |
| 50% | 9.24 (132.0) | 9.051 (129.3) | 9.296 (132.8) | 9.996 (142.8) | 9.555 (136.5) | 9.926 (141.8) |
| Tensile Strength, (psi) N/$cm^2$ | 24.8 (35.9) | 24.4 (35.4) | 21.9 (31.7) | 26.3 (38.1) | 25.6 (37.1) | 22.1 (32.1) |
| Elongation, % | 104 | 101 | 97 | 94 | 90 | 81 |
| 50% Compression Set, $\%C_T$ | 4.3 | 4.1 | 3.9 | 4.6 | 4.3 | 4.3 |
| Wet Set, % | 18.0 | 16.4 | 16.9 | 16.1 | 17.7 | 15.9 |

## Example 9

The procedure of Example 1 was repeated using the ingredients (including Additive-B) set out in Table X below. Table X shows the effects of increasing foam density via water level reductions on wet set properties. Polymer polyol is also reduced as density is increased in the effort to maintain constant load properties over the density range. However, the catalyst content remained constant in this series of runs. The data in Table X shows a strong influence of $H_2O$ level/density on foam wet set properties. As the density is increased, the wet set values are reduced significantly and the elongation values are increased significantly. Regular compression sets are also reduced in response to the increased foam density.

TABLE X

| Cushion Foam | | | | | | |
|---|---|---|---|---|---|---|
| Polymer Polyol Density Effects | | | | | | |
| Formulations | | | | | | |
| Components | Parts by Weight | | | | | |
| Polyol-E | 35 | 40 | 45 | 50 | 55 | 60 |
| Polymer-Polyol-E | 65 | 60 | 55 | 50 | 45 | 40 |
| Additive-B | 2.0 | 2.0 | 2.0 | 2.0 | 2.0 | 2.0 |
| $H_2O$ | 3.3 | 3.1 | 2.9 | 2.7 | 2.5 | 2.3 |
| DEOA | 2.0 | 2.0 | 2.0 | 2.0 | 2.0 | 2.0 |
| Catalyst-D | 0.5 | 0.5 | 0.5 | 0.5 | 0.5 | 0.5 |
| Surfactant-A | 1.0 | 1.0 | 1.0 | 1.0 | 1.0 | 1.0 |
| TDI (105 Index) | 43.1 | 41.2 | 39.2 | 37.3 | 35.4 | 33.4 |
| Foam Properties | | | | | | |
| | 43.2 | 46.4 | 49.6 | 52.8 | 56 | 60.8 |
| Density,(pcf) kg/m$^3$ | 2.7 | 2.9 | 3.1 | 3.3 | 3.5 | 3.8 |
| Resiliency % | 67 | 68 | 68 | 68 | 71 | 71 |
| kg/cm$^2$ | | | | | | |
| IFD,(lbs./50 in.) | 4.291 | 4.41 | 4.781 | 5.201 | 4.515 | 4.445 |
| 25% | (61.3) | (63.0) | (68.3) | (74.3) | (64.5) | (63.5) |
| | 8.33 | 8.47 | 8.911 | 9.751 | 8.47 | 8.015 |
| 50% | (119.0) | (121.0) | (127.3) | (139.3) | (121.0) | (114.5) |
| Tensile Strength, | 21.3 | 23.7 | 24.2 | 28.0 | 21.1 | 22.4 |
| (psi) N/cm$^2$ | (30.9) | (34.3) | (35.1) | (40.6) | (30,6) | (32.4) |
| Elongation, % | 80 | 87 | 95 | 108 | 115 | 118 |
| 50% Compression Set, %$C_T$ | 4.1 | 3.8 | 1.7 | 3.7 | 3.2 | 2.8 |
| Wet Set, % | 14.9 | 12.7 | 11.0 | 11.0 | 9.9 | 8.2 |

## Example 10

The procedure of Example 1 was repeated using the ingredients (including Additive-B) set out in Table XI below. Reducing the water level in a formulation leads to several changes in foam properties other than a density increase. The foam firmness will also increase and cure rate will be improved. Thus, it is possible to reduce the polymer polyol and catalyst levels while decreasing the water levels to maintain constant firmness and cure rate as the density is increased. The data in Table XI shows this result. The data shows that foam wet set is improved over those of Table X where the catalyst level is held constant. All of these foams exhibit 3-minute demold characteristics.

TABLE XI

| Cushion Foam | | | | | |
|---|---|---|---|---|---|
| Polymer Polyol Density Catalyst Effects | | | | | |
| Formulations | | | | | |
| Components | Parts by Weight | | | | |
| Polyol-E | 45 | 50 | 55 | 60 | 65 |
| Polymer-Polyol-E | 55 | 50 | 45 | 40 | 35 |
| Additive-B | 2.0 | 2.0 | 2.0 | 2.0 | 2.0 |
| $H_2O$ | 3.3 | 3.1 | 2.9 | 2.7 | 2.5 |
| DEOA | 1.0 | 1.0 | 1.0 | 1.0 | 1.0 |
| Catalyst-D | 0.5 | 0.45 | 0.4 | 0.35 | 0.3 |
| Surfactant-A | 1.0 | 1.0 | 1.0 | 1.0 | 1.0 |
| TDI (105 Index) | 40.6 | 38.7 | 36.8 | 34.9 | 33.0 |
| Foam Properties | | | | | |
| Density, (pcf) kg/m$^3$ | 44.8 (2.8) | 49.6 (3.1) | 52.8 (3.3) | 56 (3.5) | 60.8 (3.8) |
| Resiliency % | 64 | 64 | 67 | 67 | 69 |
| kg/cm$^2$ | | | | | |
| IFD,(lbs./50 in.) 25% | 5.516 (78.8) | 6.181 (88.3) | 6.3 (90.0) | 6.216 (88.8) | 4.865 (96.5) |
| 50% | 9.751 (139.3) | 10.815 (154.5) | 11.011 (157.3) | 10.731 (153.3) | 11.563 (164.8) |
| Tensile Strength, (psi) N/cm$^2$ | 22.1 (32.1) | 23.2 (33.6) | 22.2 (32.2) | 19.6 (28.4) | 21.3 (30.8) |
| Elongation, % | 95 | 95 | 101 | 104 | 106 |
| 50% Compression Set, %$C_T$ | 3.4 | 3.3 | 3.1 | 3.2 | 2.6 |
| Wet Set, % | 12.5 | 10.9 | 8.9 | 7.1 | 6.4 |

## Example 11

The procedure of Example 1 was repeated using the ingredients (including Additive-B) set out in Table XIII below. Table XII shows the effects of varying the polymer/polyol in a low water/high density foam formulation where the amine catalyst system is held constant at a very low level. The demold times of these foams are around seven minutes each. Wet set values are very low in every foam due to the high density and low catalyst level. The polymer/polyol level is seen to have very little effect upon any foam properties, except the loads and tensile strength properties.

TABLE XII

| Cushion Foam | | | | | |
|---|---|---|---|---|---|
| Polymer Polyol High Density Low Catalyst Effects | | | | | |
| Formulations | | | | | |
| Components | Parts by Weight | | | | |
| Polyol-E | 50 | 55 | 60 | 65 | 70 |
| Polymer-Polyol-E | 50 | 45 | 40 | 35 | 30 |
| Additive-B | 2.0 | 2.0 | 2.0 | 2.0 | 2.0 |
| $H_2O$ | 2.5 | 2.5 | 2.5 | 2.5 | 2.5 |
| DEOA | 1.0 | 1.0 | 1.0 | 1.0 | 1.0 |
| Catalyst-A | 0.075 | 0.075 | 0.075 | 0.075 | 0.075 |
| Catalyst-C | 0.225 | 0.225 | 0.225 | 0.225 | 0.225 |
| Surfactant-A | 1.0 | 1.0 | 1.0 | 1.0 | 1.0 |
| TDI (105 Index) | 32.7 | 32.8 | 32.9 | 33.0 | 33.1 |
| Foam Properties | | | | | |
| | 60.8 | 60.8 | 60.8 | 60.8 | 60.8 |
| Density, (pcf)kg/m$^3$ | 3.8 | 3.8 | 3.8 | 3.8 | 3.8 |
| Resiliency % | 67 | 69 | 70 | 72 | 72 |
| kg/cm$^2$ | | | | | |
| IFD,(lbs./50 in.) | 7.399 | 6.90 | 6.643 | 5.999 | 6.251 |
| 25% | (105.7) | (99.0) | (94.9) | (85.7) | (89.3) |
| | 12.901 | 12.075 | 11.515 | 10.395 | 10.766 |
| 50% | (184.3) | (172.5) | (164.5) | (148.5) | (153.8) |
| Tensile Strength, | 24.3 | 21.9 | 20.2 | 19.2 | 17.6 |
| (psi) N/cm$^2$ | (35.2) | (31.8) | (29.3) | (27.9) | (25.5) |
| Elongation, % | 104 | 89 | 110 | 110 | 91 |
| 50% Compression Set,%$C_T$ | 2.4 | 2.4 | 2.5 | 2.4 | 2.2 |
| Wet Set, % | 7.0 | 6.4 | 6.9 | 6.6 | 6.5 |

## Claims

1. A process for the preparation of high resilience polyurethane foam, by using a composition comprising:

(a) at least one ethylene oxide adduct of a trifunctional starter compound or a difunctional starter compound;

(b) at least one polyether polyol, provided that the polyether polyol is not the same as the adduct (a);

(c) at least one catalyst;

(d) water;

(e) at least one foam stabilizer;

(f) at least one compound containing at least two isocyanate reactive groups; and

(g) a blowing agent.

2. The process as claimed in Claim 1 wherein the ethylene oxide adduct of a trifunctional starter compound is an ethylene oxide adduct of glycerine.

3. The process as claimed in Claim 2 wherein the ethylene oxid adduct of glycerine has an average molecular weight of 500 to 10,000, preferably of about 1000.

4. The process as claimed in Claims 1 - 3 wherein the starter compound is a diol, or a triol, or a primary amine, or an amino alcohol with no more than three active hydrogens.

5. The process as claimed in Claims 1 - 4 wherein the polyether polyol has an average molecular weight of greater than 4000.

6. The process as claimed in Claims 1 - 5 wherein stabilizer (e) is a silicone surfactant.

7. The process as claimed in Claim 6 wherein a further reactive stabilizer is present.

8. The process as claimed in Claim 1 wherein a chain extender is also present.

9. The process as claimed in Claims 1 - 8 wherein the compound containing isocyanate reactive groups has the formula:

$R(NCO)_z$

wherein R is a polyvalent organic radical which is either aliphatic, aralkyl, alkaryl, aromatic or mixtures thereof, and z is an integer which corresponds to the valence of R and is a least two.

10. The process as claimed in Claims 8 and 9 wherein the compound containing at least two isocyanate reactive groups is tolylenediisocyanate, polymethylene polyphenyl isocyanate and mixtures thereof.

11. The process as claimed in Claims 1 - 10 wherein the reaction step is conducted at a temperature of 5 to 68 °C.

12. The process as claimed in Claim 11 wherein the composition is preheated to a temperature of 21 to 27 °C before the reaction step is conducted.

13. High resilience polyurethane foam articles prepared by the process of claims 1 - 12 as slab stock polyurethane foam articles and high resilience molded polyurethane foam articles in automotive cushion or seat form.